# EUROPÄISCHE PATENTSCHRIFT

(11) **EP 1 490 507 B1**
(45) Veröffentlichungstag und Bekanntmachung des Hinweises auf die Patenterteilung: **13.12.2006**
(21) Anmeldenummer: 03745279.4
(22) Anmeldetag: 27.03.2003
(51) Int. Cl.: C12Q 1/02, C12Q 1/68

(54) **VERFAHREN ZUM NACHWEIS VON MIKROORGANISMEN MITTELS IN SITU-HYBRIDISIERUNG UND DURCHFLUSSZYTOMETRIE**
METHOD FOR THE IDENTIFICATION OF MICROORGANISMS BY MEANS OF IN SITU HYBRIDIZATION AND FLOW CYTOMETRY
PROCEDE DE DETECTION DE MICRO-ORGANISMES PAR UNE HYBRIDATION IN SITU ET UNE CYTOMETRIE DE FLUX

(30) Priorität: 28.03.2002 DE 10214153
(43) Veröffentlichungstag der Anmeldung: 29.12.2004
(73) Patentinhaber: Vermicon AG, 80992 München (DE)
(72) Erfinder: SNAIDR, Jiri, 80992 München (DE); BEIMFOHR, Claudia, 80992 München (DE); THELEN, Karin, 80992 München (DE)
(74) Vertreter: Bohmann, Armin K.
(86) Internationale Anmeldenummer: PCT/EP2003/003204
(87) Internationale Veröffentlichungsnummer: WO 2003/083131

(56) Entgegenhaltungen:
- DE-A- 19 945 553
- WALLNER G ET AL: "OPTIMIZING FLUORESCENT IN SITU HYBRIDIZATION WITH RRNA-TARGETED OLIGONUCLEOTIDE PROBES FOR FLOW CYTOMETRIC IDENTIFICATION OF MICROORGANISMS" CYTOMETRY, ALAN LISS, NEW YORK, US, Bd. 14, Nr. 2, 1993, Seiten 136-143, XP000916755 ISSN: 0196-4763
- RIGOTTIER-GOIS LIONEL ET AL: "Fluorescent hybridisation combined with flow cytometry and hybridisation of total RNA to analyse the composition of microbial communities in human faeces using 16S rRNA probes." FEMS MICROBIOLOGY ECOLOGY, Bd. 43, Nr. 2, 2003, Seiten 237-245, XP002248855 ISSN: 0168-6496
- THOMAS JEAN-CHRISTOPHE ET AL: "Quantitative flow cytometric detection of specific microorganisms in soil samples using rRNA targeted fluorescent probes and ethidium bromide." CYTOMETRY, Bd. 27, Nr. 3, 1997, Seiten 224-232, XP009014220 ISSN: 0196-4763
- BREHM-STECHER B F ET AL: "Combined Fluorescence In Situ Hybridization and tetrazolium-based viability estimation of Salmonella and Listeria monocytogenes." ABSTRACTS OF THE GENERAL MEETING OF THE AMERICAN SOCIETY FOR, Bd. 101, 2001, Seite 574 XP009014219 101st General Meeting of the American Society for Microbiology;Orlando, FL, USA; May 20-24, 2001, http://www.asmusa.org/mtgsrc/generalmeetin g.htm 2001 ISSN: 1060-2011
- AMANN R I ET AL: "PHYLOGENETIC IDENTIFICATION AND IN SITU DETECTION OF INDIVIDUAL MICROBIAL CELLS WITHOUT CULTIVATION" MICROBIOLOGICAL REVIEWS, AMERICAN SOCIETY FOR MICROBIOLOGY, WASHINGTON, DC, US, Bd. 59, Nr. 1, 1. März 1995 (1995-03-01), Seiten 143-169, XP002026194 ISSN: 0146-0749 in der Anmeldung erwähnt
- WALLNER GUENTER ET AL: "Flow sorting of microorganisms for molecular analysis." APPLIED AND ENVIRONMENTAL MICROBIOLOGY, Bd. 63, Nr. 11, November 1997 (1997-11), Seiten 4223-4231, XP002248856 ISSN: 0099-2240 in der Anmeldung erwähnt

## Beschreibung

Die Erfindung betrifft ein kombiniertes Verfahren zum spezifischen Nachweis von Mikroorganismen durch in situ-Hybridisierung und Durchflusszytometrie. Das Verfahren zeichnet sich vor allem durch eine gegenüber dem Stand der Technik verbesserte Spezifität und eine kürzere Verfahrensdauer aus.

Traditionell erfolgt der Nachweis von Mikroorganismen mittels Kultivierung. Dieses Nachweisverfahren weist aber eine Reihe von Nachteilen auf. Insbesondere bei Analyse der Biozönose von Umweltproben hat sich die Kultivierung als gänzlich ungeeignet erwiesen. Kultivierungsabhängige Verfahren liefern nur einen sehr verfälschten Einblick in die Zusammensetzung und Dynamik der mikrobiellen Biozönose. So konnte gezeigt werden, dass es z.B. bei der Erfassung der Belebtschlammflora durch Kultivierung zu einem Kultivierungsshift kommt (Wagner, M., R. Amann, H. Lemmer, and K.H. Schleifer. 1993. Probing activated sludge with oligonucleotides specific for proteobacteria: inadequacy of culturedependent methods for describing microbial community structure. Appl. Environ. Microbiol. 59:1520-1525).

Durch diese mediumabhängige Verzerrung der realen Verhältnisse innerhalb der Bakterienpopulation werden Bakterien, die im Belebtschlamm eine untergeordnete Rolle spielen, aber den verwendeten Kultivierungsbedingungen gut angepasst sind, in ihrer Bedeutung dramatisch überschätzt. So konnte gezeigt werden, dass aufgrund eines solchen Kultivierungsartefaktes die Bakteriengattung *Acinetobacter* bezüglich ihrer Rolle als biologischer Phosphatentferner in der Abwasserreinigung völlig falsch eingeschätzt wurde. Als Folge solcher Fehlbewertungen entstehen kostenintensive, fehlerbehaftete bzw. unpräzise Modellierungen von Anlagen. Die Effizienz und Reproduzierbarkeit solcher Simulationsberechnungen ist gering.

Aber auch bei der Analyse von Lebensmittel- oder medizinischen Proben weist die Kultivierung bedeutende Nachteile auf. Die hier verwendeten Verfahren sind oft sehr langwierig, erfordern eine Vielzahl aufeinander folgender Kultivierungsschritte und sind nicht selten in ihrem Ergebnis nicht eindeutig. Als Beispiel sei hier die Untersuchung einer Wasserprobe auf die An- oder Abwesenheit von Fäkalstreptokokken beschrieben. Die in der deutschen Trinkwasserverordnung empfohlenen Nachweisverfahren basieren auf direkter Kultivierung der Wasserprobe oder auf Membranfiltration und anschließendem Einbringen des Filters in 50 ml Azid-Glucose-Bouillon. Die Kultivierung soll für mindestens 24 h, bei negativem Ergebnis für 48 h, bei 36 °C erfolgen. Ist auch nach 48 h keine Trübung oder Sedimentbildung der Bouillon feststellbar, gilt die Abwesenheit von Fäkalstreptokokken in der untersuchten Probe als belegt. Im Falle einer Trübung oder Sedimentbildung erfolgt ein Ausstrich der Kultur auf Enterokokken-Selektivagar nach Slanetz-Barthley und die erneute Inkubation bei 36 °C für 24 h. Im Falle der Bildung rotbrauner bzw. rosafarbener Kolonien werden diese genauer untersucht. Nach Überführung in ein geeignetes Flüssigmedium und Kultivierung für 24 h bei 36 °C, gelten Fäkalstreptokokken als nachgewiesen, wenn eine Vermehrung in Nährbouillon bei pH 9,6 erfolgt und Vermehrung in 6,5 % NaCl-Bouillon möglich ist sowie bei Äsculinabbau. Der Äsculinabbau wird durch Zugabe von frisch hergestellter 7%iger wässriger Lösung von Eisen(II)-chlorid zur Äsculinbouillon geprüft. Bei Abbau entsteht eine braun-schwarze Farbe. Häufig wird zusätzlich eine Gramfärbung zur Unterscheidung der Bakterien von Gram-negativen Kokken durchgeführt sowie ein Katalasetest zur Unterscheidung von Staphylokokken durchgeführt. Fäkalstreptokokken reagieren Gram-positiv und Katalase-negativ. Der traditionelle Nachweis stellt sich somit als langwieriges (48 -100 h) und im Verdachtfall überaus aufwendiges Verfahren dar.

Aufgrund der beschriebenen Nachteile der Kultivierung haben moderne Methoden der Bakterienbestimmung alle ein gemeinsames Ziel: Sie versuchen die Nachteile der Kultivierung zu umgehen, indem sie keine Kultivierung der Bakterien mehr benötigen oder doch zumindest die Kultivierung auf ein Minimum reduzieren.

Bei der PCR, der Polymerase-Kettenreaktion wird mit bakterienspezifischen Primern ein bestimmtes charakteristisches Stück des Bakteriengenoms amplifiziert. Findet der Primer seine Zielstelle, so kommt es zu einer millionenfachen Vermehrung eines Stücks der Erbsubstanz. Bei der anschließenden Analyse mittels eines DNA-Fragmente auftrennenden Agarose-Gels kann eine qualitative Bewertung stattfinden. Im einfachsten Fall führt dies zu der Aussage, dass die Zielstellen in der untersuchten Probe vorhanden sind. Keine weiteren Aussagen sind zulässig, da die Zielstellen von einem lebenden Bakterium, von einem toten Bakterium oder von nackter DNA stammen können. Eine Differenzierung ist hier nicht möglich. Eine Weiterführung dieser Technik stellt die quantitative PCR dar, bei der versucht wird, eine Korrelation zwischen Menge an vorhandenen Bakterien und Menge an erhaltener und amplifizierter DNA herzustellen. Allerdings können diverse in der analysierten Probe enthaltene Stoffe eine Inhibierung des DNA amplifizierenden Enzyms, der Taq-Polymerase, herbeiführen. Dies ist eine häufige Ursache falsch negativer Ergebnisse der PCR. Vorteile der PCR liegen in ihrer hohen Spezifität, leichten Anwendbarkeit und im geringen Zeitaufwand. Wesentliche Nachteile sind ihre hohe Anfälligkeit für Kontaminationen und damit falsch positive Ergebnisse sowie die bereits erwähnte fehlende Möglichkeit zwischen lebenden und toten Zellen bzw. nackter DNA zu unterscheiden und schließlich die Gefahr falsch negativer Ergebnisse infolge der Anwesenheit inhibitorischer Substanzen.

### Doch auch biochemische Parameter werden zur Bakterienidentifizierung verwendet:

So dient die Erstellung von Bakterienprofilen auf der Basis von Quinonbestimmungen dazu, ein möglichst verzerrungsfreies Bild der Bakterienpopulation wiederzugeben (Hiraishi, A. 1988. Respiratory quinone profiles as tools for identifying different bacterial populations in activated sludge. J. Gen. Appl. Microbiol. 34:39-56). Doch auch diese Methode ist von der Kultivierung einzelner Bakterien abhängig, da zur Erstellung der Referenzdatenbank die Quinonprofile der Bakterien in Reinkultur benötigt werden. Überdies vermag die Bestimmung der Bakterienquinonprofile auch keinen wirklichen Eindruck davon zu vermitteln, welche tatsächlichen Populationsverhältnisse in der Probe vorliegen.

Die Detektion der Bakterien mit Antikörpern ist im Unterschied hierzu eine direktere Methode (Brigmon, R. L., G. Bitton, S. G. Zam, and B. O'Brien. 1995. Development and application of a monoclonal antibody against Thiothrix spp. Appl. Environ. Microbiol. 61:13-20). Fluoreszenzmarkierte Antikörper werden mit der Probe vermengt und erlauben ein hochspezifisches Anheften an die bakteriellen Antigene. Im Epifluoreszenz-Mikroskop werden die so markierten Bakterien anhand ihrer emittierten Fluoreszenz anschließend detektiert. Auf diese Weise können Bakterien bis auf Stammebene identifiziert werden. Entscheidende Nachteile schränken aber die Anwendbarkeit dieser Methode empfindlich ein: Erstens werden für die Herstellung der Antikörper zunächst einmal Reinkulturen der nachzuweisenden Bakterien benötigt. Dies bedeutet natürlich, dass letztendlich nur solche Bakterien, die sich überhaupt kultivieren lassen, mit Antikörpern nachzuweisen sind. Der größte Teil der Bakterien ist aber nicht kultivierbar und kann daher mit diesem Verfahren nicht nachgewiesen werden. Zweitens bereitet der oftmals großvolumige und sperrige Antikörper-Fluoreszenzmolekülkomplex Probleme beim Eindringen in die Zielzellen. Drittens ist die Anwendung von Antikörpern auf bestimmte, in geeigneter Form vorliegende oder entsprechend aufbereitete Proben beschränkt. Gerade Umweltproben, die häufig einen hohen Anteil an partikulärem Material aufweisen (z.B. Bodenproben oder Klärschlammproben) lassen sich nur unzureichend mittels Antikörpern analysieren. In diesen Proben kommt es verstärkt zur unspezifischen Adsorption der Antikörper an die enthaltenen Partikel. Dies kann zu falsch-positiven Ergebnissen führen, wenn die fluoreszierenden Partikel mit den nachzuweisenden Bakterien verwechselt werden. Zumindest ist aber die Auswertung der Analyse sehr erschwert, da unspezifisch leuchtende Partikel von spezifisch leuchtenden Bakterien unterschieden werden müssen. Viertens ist der Nachweis mittels Antikörpern oftmals zu speziell. Die Antikörper weisen oftmals hochspezifisch nur einen bestimmten Bakterienstamm einer Bakterienart nach, lassen aber andere Stämme der gleichen Bakterienart unentdeckt. In den meisten Fällen ist aber gerade keine stammspezifische Detektion der Bakterien erforderlich, sondern vielmehr die Detektion aller Bakterien einer Bakterienart oder einer ganzen Bakteriengruppe. Für viele Bakterienarten ist dies bislang nicht gelungen: ein auf Antikörpern basierendes Nachweisverfahren zu entwickeln, welches nicht nur einzelne Stämme, sondern alle Bakterien der Art erfasst. Fünftens ist die Produktion der Antikörper ein relativ langwieriger und teurer Vorgang.

Als neuartiger Ansatz wurde Anfang der neunziger Jahre das Verfahren der in situ-Hybridisierung mit fluoreszenzmarkierten Oligonukleotidsonden entwickelt, die sogenannte Fluoreszenz-In-Situ-Hybridisierung (FISH; Amann et al. (1990) J. Bacteriol. 172:762; Amann, R. I., W. Ludwig, and K.-H. Schleifer. 1995. Phylogenetic identification and in situ detection of individual microbial cells without cultivation. Microbial. Rev. 59:143-169). Hierbei können Bakterienarten, -gattungen oder -gruppen hochspezifisch direkt in der Probe identifiziert und bei Bedarf auch visualisiert oder quantifiziert werden. Diese Methode ist das einzige Verfahren, das eine verzerrungsfreie Darstellung der tatsächlichen in situ-Verhältnisse der Biozönose darstellt. Sogar bisher nicht-kultivierte und demnach nicht beschriebene Bakterien können identifiziert werden.

Die FISH-Technik basiert auf der Tatsache, dass es in Bakterienzellen bestimmte Moleküle gibt, die aufgrund ihrer lebenswichtigen Funktion im Laufe der Evolution nur wenig mutiert wurden: Die 16S und die 23S ribosomale Ribonukleinsäure (rRNS). Beide sind Bestandteile der Ribosomen, den Orten der Proteinbiosynthese, und können aufgrund ihrer ubiquitären Verbreitung, ihrer Größe, und ihrer strukturellen und funktionellen Konstanz als spezifische Marker dienen (Woese, C. R. 1987. Bacterial evolution. Microbiol. Rev. 51:221-271).

Für die Anwendung der FISH werden so genannte Gensonden (i.d.R. kleine, 16-25 Basen lange, einzelsträngige Desoxyribonukleinsäurestücke) entwickelt, die zu einer definierten Region der rRNA komplementär sind. Diese definierte Region wird jeweils so ausgewählt, dass sie spezifisch ist für eine Bakterienart, -gattung oder -gruppe.

Bei der FISH dringen markierte Gensonden in die in der untersuchten Probe vorhandenen Zellen ein. Sofern ein Bakterium der Art, Gattung oder Gruppe, für welche die Gensonden entwickelt wurden, in der untersuchten Probe vorhanden ist, binden die Gensonde in der Bakterienzelle an ihre Zielsequenz, und die Zellen können aufgrund der Markierung der Gensonden detektiert werden.

Die Vorteile der FISH-Technik gegenüber den weiter oben beschriebenen Methoden zur Bakterienidentifizierung (Kultivierung, PCR oder Antikörper) sind vielfältig.

Erstens können mit Gensonden zahlreiche Bakterien detektiert werden, die mittels traditioneller Kultivierung gar nicht erfasst werden. Während durch die Kultivierung maximal nur 15 % der Bakterienpopulation einer Probe sichtbar gemacht werden können, erlaubt die FISH-Technik in vielen Proben eine Detektion bis zu 100 % der Bakteriengesamtpopulation. Zweitens ist die Detektion von Bakterien mittels der FISH-Technik sehr viel schneller als mittels Kultivierung. Benötigt die Identifizierung von Bakterien mittels Kultivierung oft mehrere Tage, so vergehen von der Probennahme bis zur Bakterienidentifizierung sogar auf Artebene mittels der FISH-Technik nur wenige Stunden. Drittens können im Gegensatz zu einem Kultivierungsmedium Gensonden in ihrer Spezifität fast beliebig frei gewählt werden. Einzelne Arten können genauso gut mit einer Sonde erfasst werden, wie ganze Gattungen oder Bakteriengruppen. Viertens können Bakterienarten oder ganze Bakterienpopulationen direkt in der Probe exakt quantifiziert werden.

Im Gegensatz zur PCR kann die FISH zuverlässig nur lebende Bakterien nachweisen. Falsch positive Ergebnisse durch nackte DNA oder tote Bakterien wie bei der PCR sind bei der FISH ausgeschlossen. Des weiteren sind falsch negative Ergebnisse infolge der Anwesenheit inhibitorischer Substanzen ebenso ausgeschlossen wie falsch positive Ergebnisse infolge von Kontaminationen.

Im Gegensatz zur Antikörpertechnologie ist die Produktion der Nukleinsäuresondenmoleküle einfach, schnell und preisgünstig zu bewerkstelligen. Des weiteren ist der Komplex aus Nukleinsäuresondenmolekül und Fluoreszenzfarbstoff bei weitem kleiner als der Antikörper-Farbstoff-Komplex und kann im Gegensatz zu letzterem leicht in die nachzuweisenden Zellen eindringen. Auch kann wie bereits weiter oben beschrieben, die Spezifität der Nukleinsäuresondenmoleküle fast beliebig frei gewählt werden. Einzelne Arten können genauso gut mit einer Sonde erfasst werden, wie ganze Gattungen oder Bakteriengruppen. Schließlich ist im-Gegensatz-zur Antikörpertcehnologie die FISH-Technik zur Untersuchung verschiedenster Probenarten geeignet

Die FISH-Technik ist demnach ein überragendes Werkzeug, um Bakterien schnell und äußerst spezifisch direkt in einer Probe nachzuweisen. Sie ist im Gegensatz zu Kultivierungsverfahren eine direkte Methode und erlaubt darüber hinaus auch eine Quantifizierung.

Standardmäßig wird die FISH-Analyse auf einem geeigneten festen lichtdurchlässigen Träger, wie z.B. einem Objektträger oder einer Mikrotiterplatte, durchgeführt. Die Auswertung erfolgt dann mittels eines Mikroskops, wobei die Bakterien durch Bestrahlung mit einem hochenergetischen Licht visualisiert, also sichtbar gemacht werden.

Das herkömmliche FISH-Verfahren zum Nachweis von Mikroorganismen unter Verwendung eines festen Trägers weist jedoch durchaus Limitationen auf. Es ist nicht oder nur schwer automatisierbar und daher vergleichsweise langwierig, außerdem nicht immer mit gleicher Qualität reproduzierbar. Außerdem gilt als mögliche Fehlerquelle bei der quantitativen Analyse am Mikroskop die nie gänzlich zu eliminierende Subjektivität des Betrachters.

Vor allem die fehlende Automatisierbarkeit und die somit vergleichsweise aufwendige, und langwierige Hand(!)habung sowie die dem subjektiven Eindruck des Betrachters unterworfene Quantifizierung haben dazu geführt, dass die FISH-Analyse bislang in der Industrie lediglich für Einzel- und Mehrfachanalysen, nicht jedoch für Hochdurchsatzanalysen verwendet und nur selten zur exakten Quantifizierung eingesetzt wird. Da die Analyse von Bakterien mittels dieses Verfahrens jedoch, wie beschrieben, bedeutende Vorteile gegenüber allen anderen heutzutage industriell verwendeten mikrobiologischen Analyseverfahren besitzt, besteht ein Bedarf an einem kombinierten Verfahren, welches die Vorteile der FISH-Analyse mit denen eines schnellen, einfachen, automatisierbaren und objektiven Verfahrens verknüpft und das somit die einfache, schnelle und zuverlässige Analyse von Proben mit dem Zweck der Identifizierung und Quantifizierung von Mikroorganismen ermöglicht.

Als "automatisierte Mikroskopie" hat die Durchflusszytometrie in den letzten Jahren vor allem in der biologischen und medizinischen Forschung sowie in der Diagnostik verstärkt an Einfluss gewonnen. Sie bietet die Vorteile der Automatisierung, Objektivität und einer hohen Auswertegeschwindigkeit (es können mehrere 1000 Zellen pro Sekunde vermessen werden).

Die Durchflusszytometrie ermöglicht das Zählen und die Analyse von physikalischen und molekularen Eigenschaften von Partikeln (wie z.B. Zellen) in einem Flüssigkeitsstrom. Die Durchflusszytometrie ermöglicht die Dokumentation bestimmter Eigenschaften von Zellen oder Zellpopulationen auf Einzelzellebene.

Das Durchflusszytometer besteht prinzipiell aus einem Flüssigkeits- und einem optischen System. Die Basis für eine erfolgreiche Untersuchung ist die hydrodynamische Fokussierung der Probe durch das Flüssigkeitssystem. Dabei werden die in der Probe enthaltenen Zellen vereinzelt und mit einer sehr hohen Genauigkeit (Abweichung 1 µm) linear am Messpunkt angeordnet. Das optische System besteht generell aus einer Anregungslichtquelle (z.B. diverse Laser oder eine Quecksilberdrucklampe), verschiedenen optischen Spiegeln und Detektoren für das Vorwärtsstreulicht, das Seitwärtsstreulicht und das Fluoreszenzlicht. Durchflusszytometer sind bereits von mehreren Anbietern erhältlich, wie z.B. das Produkt Microcyte von Optoflow, Norwegen, oder das Gerät BD FACSCalibur von BD Biosciences, Becton, Dickinson and Company. Darüber hinaus bieten Firmen und Institute die Durchführung entsprechender Durchflusszytometrie-Analysen bzw. Nutzungszeiten für Durchflusszytometer an.

Zu Beginn der Analyse wird die Probe mittig in die Transportflüssigkeit eingeführt. Die Vereinzelung und Zentrierung der Zellen erfolgt durch einen Hüll- bzw. Mantelstrom der durch eine höhere Fließgeschwindigkeit der Transportflüssigkeit gegenüber der des Probenstroms erzeugt wird. Durch das unter Druck stehende Flüssigkeitssystem werden nun die vereinzelten Zellen kontinuierlich und mit konstanter Geschwindigkeit an der Anregungslichtquelle des optischen Systems vorbeigeführt.

Das auf die Zellen auftreffende Laserlicht wird zunächst in zwei Richtungen gestreut: das nach "vorne", in einem Winkel von 2 -15 ° (FSC für Forwardscatter) und das "zur Seite", in einem Winkel von 15-90° (SSC für Sidewardscatter) abgelenkte Streulicht ist ein Maß für Größe und Granularität der Zelle. Zusätzlich können über diverse Laser (z.B. Argon- oder Helium-Neon-Laser) oder eine Quecksilberdrucklampe mit entsprechenden optischen Filtern verschiedene Fluorochrome zur Emittierung von Lichtquanten angeregt werden. Diese werden dann schließlich von den entsprechenden Sensoren detektiert. Die erhaltenen Messwerte werden in Form von Histogrammen oder Dotplots auf dem Computer visualisiert.

Bislang wird die Durchflusszytometrie allerdings nur in sehr geringem Umfang für mikrobiologische Untersuchungen eingesetzt. Erste Versuche, die FISH und die Durchflusszytometrie miteinander zu verknüpfen, stammen von Wallner (Wallner G. et al. (1993) Cytometry 14(2):136-143; Wallner G. et al. (1995) Appl. Environ. Microbiol. 61(5):1859-1866; Wallner G. et al. (1997) Appl. Environ. Microbiol. 63(11):4223-4231).

Nachteile des im Stand der Technik beschriebenen Verfahrens sind die wiederum recht langwierige Durchführung (Hybridisierungsdauer von 3 Stunden, Zentrifugationsschritt zwischen Hybridisierung und Waschen, Waschdauer von 0,5 Stunden), die infolge dieser langwierigen Durchführung nicht sicher gewährleistete Spezifität des Verfahrens sowie die fehlende Eignung dieses Verfahrens zum Nachweis grampositiver Bakterien.

Aufgabe der vorliegenden Erfindung ist es, die vorstehend beschriebenen Nachteile des Standes der Technik zu überwinden und ein Verfahren bereitzustellen, mit dem der spezifische Nachweis von Mikroorganismen einfach, reproduzierbar, zuverlässig, schnell und objektiv durchgeführt werden kann.

Die oben genannte Aufgabe wird erfindungsgemäß durch die Merkmale des unabhängigen Anspruchs gelöst. Weitere Ausführungsformen ergeben sich aus den Merkmalen der Unteransprüche.

Die Durchführung des erfindungsgemäßen Verfahrens zum spezifischen Nachweis von Mikroorganismen in einer Probe umfasst die folgenden Schritte:
a) Fixieren der in der Probe enthaltenen Mikroorganismen,
b) Inkubieren der fixierten Mikroorganismen mit in einer Hybridisierungslösung enthaltenen Nukleinsäuresondenmolekülen, um eine Hybridisierung herbeizuführen (= Hybridisierungsschritt),
c) Zugeben einer Waschlösung zu den fixierten und mit den Nukleinsäuresondenmolekülen inkubierten Mikroorganismen (= Waschschritt),
d) Detektieren der Mikroorganismen mit hybridisierten Nukleinsäuresondenmolekülen mittels Durchflusszytometrie,
wobei zwischen dem Hybridisierungsschritt b) und dem Waschschritt c) kein Entfernen der Hybridisierungslösung stattfindet.

In einer bevorzugten Ausführungsform umfasst das Verfahren des weiteren zwischen dem Fixierschritt a) und dem Hybridisierungsschritt b) den Schritt
i) Trocknen der Probe und Entfernen des Fixierungsmittels.

In einer weiteren bevorzugten Ausführungsform umfasst das erfindungsgemäße Verfahren weiter zwischen dem Fixierschritt a) und dem Hybridisierungsschritt b) bzw. zwischen dem Trocknungsschritt i) und dem Hybridisierungsschritt b) den Schritt: ii) Aufschließen der fixierten Mikroorganismen.

Eine besonders bevorzugte Ausführungsform des Verfahrens zum spezifischen Nachweis von Mikroorganismen in einer Probe sieht daher folgende Schritte vor:
a) Fixieren der in der Probe enthaltenen Zellen,
   i) Trocknen der Probe und Entfernen des Fixierungsmittels,
   ii) Vollständiger Aufschluss der in der Probe enthaltenen Zellen,
b) Inkubieren der fixierten und aufgeschlossenen Zellen mit Nukleinsäuresondenmolekülen, um eine Hybridisierung herbeizuführen,
c) Zugeben einer Waschlösung,
d) Detektieren der Zellen mit hybridisierten Nukleinsäuresondenmolekülen im Durchflusszytometer,
wobei zwischen Schritt b) und c) kein Entfernen der die Nukleinsäuresondenmoleküle enthaltenen Hybridisierungslösung erfolgt.

Gegebenfalls geht dem ersten Schritt eine Kurzkultivierung zur Anreicherung der in der zu untersuchenden Probe enthaltenen Zellen voraus.

In einer weiteren Ausführungsform kommt das Verfahren zudem ohne eine Zentrifugation nach dem Waschschritt aus. Durch den Verzicht jeglicher Zentrifugation kann das erfindungsgemäße Verfahren noch schneller und einfacher durchgeführt werden.

Im Rahmen der vorliegenden Erfindung wird unter "Fixieren" der Mikroorganismen eine Behandlung verstanden, mit der die Bakterienhülle für Nukleinsäuresonden durchlässig gemacht wird. Zur Fixierung wird üblicherweise Ethanol verwendet. Kann die Zellwand mit diesen Maßnahmen nicht von den Nukleinsäuresonden penetriert werden, so sind dem Fachmann ausreichend weitere Maßnahmen bekannt, die zu demselben Ergebnis führen. Dazu zählen beispielsweise Methanol, Mischungen von Alkoholen, eine niederprozentige Paraformaldehydlösung oder eine verdünnte Formaldehydlösung oder ähnliches.

Im Rahmen der vorliegenden Erfindung wird unter "Trocknen" ein Abdampfen der Probe bei erhöhter Temperatur, so lange, bis das Fixierungsmittel vollständig verdunstet ist, verstanden.

Im Rahmen der vorliegenden Erfindung wird unter "vollständigem Aufschluss der Zellen" eine enzymatische Behandlung der Zellen verstanden. Durch diese Behandlung wird die Zellwand gram-positiver Bakterien für die Nukleinsäuresondenmoleküle durchlässig gemacht. Hierfür geeignet ist beispielsweise Lysozym in einer Konzentration von 0,1-10 mg/ml H₂O. Aber auch andere Enzyme, wie beispielsweise Mutanolysin oder Proteinase K können einzeln oder in Kombination verwendet werden. Geeignete Konzentrationen und Lösungsmittel sind dem Fachmann wohlbekannt. Es versteht sich von selbst, dass das erfindungsgemäße Verfahren auch zur Analyse gram-negativer Bakterien geeignet ist; die enzymatische Behandlung zum vollständigen Zellaufschluss wird dann entsprechend adaptiert, es kann auf diese dann auch ganz verzichtet werden.

Im Rahmen der vorliegenden Erfindung werden für die "Hybridisierung" die fixierten Bakterien mit fluoreszenzmarkierten Nukleinsäuresondenmolekülen inkubiert. Diese Nukleinsäuresondenmoleküle, die aus einem Oligonukleotid und einem daran gebundenen Marker bestehen, können dann die Zellhülle penetrieren und sich an die dem Nukleinsäuresondenmolekül entsprechenden Zielsequenz im Zellinneren binden. Die Bindung ist als Ausbildung von Wasserstoffbrücken zwischen komplementären Nukleinsäurestücken zu verstehen.

Das Nukleinsäuresondenmolekül kann dabei komplementär zu einer chromosomalen oder episomalen DNA sein, aber auch zu einer mRNA oder rRNA des nachzuweisenden Mikroorganismus. Von Vorteil ist es, ein Nukleinsäuresondenmolekül zu wählen, das zu einem Bereich komplementär ist, der in einer Kopiezahl von mehr als 1 im nachzuweisenden

Mikroorganismus vorliegt. Die nachzuweisende Sequenz liegt bevorzugt 500 - 100.000 mal pro Zelle vor, besonders bevorzugt 1.000 - 50.000 mal. Aus diesem Grunde wird bevorzugt die rRNA als Zielstelle verwendet, da die Ribosomen in der Zelle als Orte der Proteinbiosynthese vieltausendfach in jeder aktiven Zelle vorliegen.

Bei dem Nukleinsäuresondenmolekül im Sinne der Erfindung kann es sich um eine DNA- oder RNA-Sonde handeln, die in der Regel zwischen 12 und 1000 Nukleotide umfassen wird, bevorzugt zwischen 12 und 500, bevorzugter zwischen 12 und 200 und zwischen 12 und 100, besonders bevorzugt zwischen 12 und 50 und zwischen 14 und 40 und zwischen 15 und 30, und am meisten bevorzugt zwischen 16 und 25 Nukleotide. Die Auswahl der Nukleinsäuresondenmoleküle geschieht nach dem Gesichtspunkt, ob eine geeignete komplementäre Sequenz in dem nachzuweisenden Mikroorganismus vorliegt. Geeignet ist eine Sequenz dann, wenn sie einerseits spezifisch für den nachzuweisenden Mikroorganismus ist und andererseits überhaupt zugänglich ist für das eindringende Nukleinsäuresondenmolekül, also nicht etwa durch ribosmale Proteine oder rRNA-Sekundärstrukturen maskiert wird. Durch diese Auswahl einer definierten Sequenz, kann dadurch eine Bakterienart, eine Bakteriengattung oder eine ganze Bakteriengruppe erfasst werden. Komplementarität sollte bei einer Sonde von 15 Nukleotiden über 100 % der Sequenz gegeben sein. Bei Oligonukleotiden mit mehr als 15 Nukleotiden sind ein bis mehrere Fehlpaarungsstellen erlaubt.

Die Nukleinsäuresondenmoleküle werden im Rahmen des erfindungsgemäßen Verfahrens mit geeigneten Hybridisierungslösungen eingesetzt. Geeignete Zusammensetzungen dieser Lösung sind dem Fachmann wohlbekannt. Eine solche Hybridisierungslösung enthält organische Lösungsmittel, insbesondere Formamid, in einer Konzentration zwischen 0 % und 80 % und hat eine Salzkonzentration (vorzugsweise NaCl) zwischen 0,1 Mol/l und 1,5 Mol/l. Weiterhin ist enthalten ein Detergens (i.d.R. SDS) in einer Konzentration zwischen 0 % und 0,2 % sowie zum Puffern der Lösung eine geeignete Puffersubstanz (z.B. Tris-HCl, Na-Citrat, HEPES, PIPES o.a.), üblicherweise in einer Konzentration zwischen 0,01 Mol/l und 0,1 Mol/l. Üblicherweise hat die Hybridisierungslösung einen pH-Wert zwischen 6,0 und 9,0.
Die Konzentration der Nukleinsäuresonde in der Hybridisierungslösung ist abhängig von der Art ihrer Markierung und der Anzahl der Zielstrukturen. Um eine schnelle und effiziente Hybridisierung zu ermöglichen, sollte die Anzahl der Nukleinsäuresondenmoleküle die Anzahl der Zielstrukturen um mehrere Größenordnungen überschreiten. Allerdings ist zu bedenken, dass eine zu hohe Menge an fluoreszenzmarkierten Nukleinsäuresondenmolekülen zu erhöhter Hintergrundfluoreszenz führt. Die Konzentration der Nukleinsäuresondenmoleküle sollte deshalb in einem Bereich zwischen 0,5 - 500 ng/µl liegen. Die im Rahmen des erfindungsgemäßen Verfahrens bevorzugte Konzentration beträgt 1-10 ng jedes verwendeten Nukleinsäuresondenmoleküls pro µl Hybridisierungslösung. Das verwendete Volumen der Hybridisierungslösung sollte zwischen 8 µl und 100 ml liegen, bei einer bevorzugten Ausführungsform des erfindungsgemäßen Verfahrens liegt es zwischen 10 µl und 1000 µl, besonders bevorzugt beträgt es zwischen 20 µl und 200 µl.

Kennzeichnend für das erfindungsgemäße Verfahren ist, dass die Konzentration und das Volumen der verwendeten Hybridisierungslösung auf das Volumen der im vorausgehenden Schritt verwendeten Enzymlösung abgestimmt werden, sofern ein enzymatischer Aufschluss stattfindet. Direkt nach dem Vermischen der Enzym- und der Hybridisierungslösung liegen die in der Hybridisierungslösung enthaltenen Chemikalien in der für die Spezifität der Nachweisreaktion erforderlichen Konzentration vor. Gleichzeitig ist die Hybridisierungslösung so zusammengesetzt, dass die Enzymreaktion zum Zellaufschluss durch die Zugabe der Hybridisierungslösung abgestoppt wird. So lässt sich die Dauer der enzymatischen Behandlung der untersuchten Probe sehr genau steuern, ohne dass ein separater Arbeitsschritt zur Entfernung der Enzymlösung durchgeführt werden muss.

Die Dauer der Hybridisierung beträgt üblicherweise zwischen zehn Minuten und zwölf Stunden; bevorzugt erfolgt die Hybridisierung für etwa 1,5 Stunden. Die Hybridisierungstemperatur liegt bevorzugt zwischen 44 °C und 48 °C, besonders bevorzugt bei 46 °C, wobei der Parameter der Hybridisierungstemperatur, wie auch die Konzentration an Salzen und Detergenzien in der Hybridisierungslösung in Abhängigkeit von den Nukleinsäuresonden, insbesondere deren Längen und dem Grad der Komplementarität zur Zielsequenz in der nachzuweisenden Zelle optimiert werden kann. Der Fachmann ist mit hier einschlägigen Berechnungen vertraut.

Erfindungsgemäß bevorzugt ist ferner, dass das Nukleinsäurensondenmolekül kovalent mit einem detektierbaren Marker verbunden ist. Dieser detektierbare Marker ist vorzugsweise ausgewählt aus der Gruppe der folgenden Marker:
- Fluoreszenzmarker,
- Chemolumineszenzmarker,
- radioaktiver Marker,
- enzymatisch aktive Gruppe,
- Hapten,
- durch Hybridisierung nachweisbare Nukleinsäure.

Besonders bevorzugt handelt es sich um einen Fluoreszenzmarker.

Im Rahmen der vorliegenden Erfindung erfolgt das "Entfernen" oder "Verdrängen" der nicht gebundenen Nukleinsäuresondenmoleküle durch Zugabe einer Waschlösung. D.h. im Unterschied zum Stand der Technik findet vor dem Waschschritt kein Entfernen der Hybridisierungslösung z.B. durch einen Zentrifugationsschritt statt. Geeignete Zusammensetzungen dieser Lösung sind dem Fachmann wohlbekannt. Diese Waschlösung kann, falls gewünscht, 0,001-0,1 % eines Detergens wie SDS sowie Tris-HCl in einer Konzentration von 0,001-0,1 Mol/l enthalten, wobei der pH-Wert von Tris-HCl im Bereich von 6,0 bis 9,0 liegt. Das Detergens kann enthalten sein, ist aber nicht zwingend erforderlich. Weiter enthält die Waschlösung üblicherweise NaCl, wobei die Konzentration je nach benötigter Stringenz von 0,003 Mol/l bis 0,9 Mol/l, bevorzugt von 0,01 Mol/l bis 0,9 Mol/l, beträgt. Des weiteren kann die Waschlösung EDTA enthalten, wobei die Konzentration vorzugsweise 0,005 Mol/l beträgt. Ferner kann die Waschlösung auch dem Fachmann geläufige Konservierungsmittel in geeigneten Mengen enthalten.

Kennzeichnend für das erfindungsgemäße Verfahren ist es, dass die Konzentration und das Volumen der verwendeten Waschlösung auf das Volumen der im vorausgehenden Schritt verwendeten Hybridisierungslösung abgestimmt werden. Direkt nach dem Vermischen der Hybridisierungslösung und der Waschlösung liegen die in der Waschlösung enthaltenen Chemikalien in der für die Spezifität der Nachweisreaktion erforderlichen Konzentration vor. Im Unterschied zu dem erfindungsgemäßen Verfahren wird im Stand der Technik zunächst die Hybridisierungslösung (z.B. durch einen Zentrifugationsschritt) entfernt und anschließend eine Waschlösung zugegeben. Hierbei sinkt die Temperatur des Reaktionsgemisches auf Raumtemperatur ab, die Folge sind unspezifische falsch positive Ergebnisse der Nachweisreaktion. Dagegen ist bei Anwendung des erfindungsgemäßen Verfahrens gewährleistet, dass die Temperatur während des gesamten Vorganges von Hybridisierung und Waschen konstant gehalten werden kann, wodurch erstmals sicher die Spezifität des Nachweisverfahrens gewährleistet ist.

Die gegenüber dem Stand der Technik überlegene Spezifität des erfindungsgemäßen Verfahrens konnte unter Verwendung verschiedener Sondenmoleküle und verschiedener Proben, also mit verschiedenen Mikroorganismen belegt werden. Die verbesserte Spezifität ist in erster Linie darauf zurückzuführen, dass kein Entfernen der Hybridisierungslösung zwischen dem Hybridisierungsschritt und dem Waschschritt erfolgt, die Waschlösung vielmehr zu den nachzuweisenden Zellen und der Hybridisierungslösung hinzugegeben wird.

Dabei wurden sehr gute Resultate erzielt, wenn das Volumen der Hybridisierungslösung 50-150 µl, besonders bevorzugt 80-120 µl betrug und die Lösung 1- bis 3-fach, besonders bevorzugt 1- bis 1,5-fach konzentriert war und das Volumen der Waschlösung 20-50 µl, besonders bevorzugt 30-40 µl betrug und die Waschlösung 3- bis 6-fach, besonders bevorzugt 4- bis 5-fach konzentriert war.

Das "Abwaschen" der nicht gebundenen Nukleinsäuresondenmoleküle erfolgt üblicherweise bei einer Temperatur im Bereich von 44 °C bis 52 °C, bevorzugt von 44 °C bis 50 °C und besonders bevorzugt bei 46 °C für eine Dauer von 10 - 40 Minuten, vorzugsweise für 15 Minuten.

Die spezifisch hybridisierten Nukleinsäuresondenmoleküle werden abschließend in den jeweiligen Zellen detektiert. Voraussetzung hierfür ist, dass das Nukleinsäuresondenmolekül nachweisbar ist, z.B. dadurch dass das Nukleinsäuresondenmolekül durch kovalente Bindung mit einen Marker verknüpft ist. Als detektierbare Marker werden z.B. fluoreszierende Gruppen wie z.B. CY2 (erhältlich von Amersham Life Sciences, Inc., Arlington Heights, USA), CY3 (ebenfalls erhältlich von Amersham Life Sciences), CY5 (ebenfalls zu beziehen von Amersham Life Sciences), FITC (Molecular Probes Inc., Eugene, USA), FLUOS (erhältlich von Roche Diagnostics GmbH, Mannheim, Deutschland), TRITC (erhältlich von Molecular Probes Inc. Eugene, USA) 6FAM oder FLUOS-PRIME verwendet, die dem Fachmann alle wohlbekannt sind. Auch chemische Marker, radioaktive Marker oder enzymatische Marker wie Meerrettich-Peroxidase, saure Phosphatase, alkalische Phosphatase, Peroxidase, können verwendet werden. Für jedes dieser Enzyme ist eine Reihe von Chromogene bekannt, die anstelle des natürlichen Substrates umgesetzt werden können, und entweder zu farbigen oder zu fluoreszierenden Produkten umgesetzt werden können. Beispiele für solche Chromogene sind in der nachfolgenden Tabelle angegeben:

**Tabelle**

| Enzyme | Chromogen |
|---|---|
| 1. Alkalische Phosphatase und saure Phosphatase | 4-Methylumbelliferylphosphat (*), Bis(4-Methyiumbelliferylphosphat), (*) 3-O-Methylfluoreszein, Flavon-3-Diphosphattriammoniumsalz (*), p-Nitrophenylphosphatdinatriumsalz |
| 2. Peroxidase | Tyraminhydrochlorid (*), 3-(p-Hydroxyphenyl)-Propionsäure (*), p-Hydroxyphenethylalkohol(*), 2,2'-Azino-di-3-ethylbenzthiazolinsulfonsäure (ABTS), ortho-Phenylendiamindihydrochlorid, o-Dianisidin, 5-Aminosalicylsäure, p-Ucresol (*), 3,3'-dimethyloxybenzidin, 3-Methyl-2-benzothiazolinhydrazon, Tetramethylbenzidin |
| 3. Meerrettichperoxidase | H₂O₂ + Diammoniumbenzidin H₂O₂ + Tetramethylbenzidin |
| 4. β-D-Galaktosidase | o-Nitrophenyl-β-D-galaktopyranosid, 4-Methylumbelliferyl-β-D-galaktosid |
| 5. Glukoseoxidase | ABTS, Glukose und Thiazolylblau |

| | |
|---|---|
| * Fluoreszenz | |

Schließlich ist es möglich, die Nukleinsäuresondenmoleküle so zu gestalten, dass an ihrem 5'- oder 3'-Ende eine weitere zur Hybridisierung geeignete Nukleinsäuresequenz vorhanden ist. Diese Nukleinsäuresequenz umfasst wiederum ca. 15 bis 1.000, bevorzugt 15-50 Nukleotide. Dieser zweite Nukleinsäurebereich kann wiederum von einem Nukleinsäuresondenmolekül erkannt werden, welches durch eines der oben erwähnten Mittel nachweisbar ist.

Eine weitere Möglichkeit besteht in der Kopplung der nachweisbaren Nukleinsäuresondenmoleküle mit einem Hapten, das anschließend mit einem das Hapten erkennenden Antikörper in Kontakt gebracht werden kann. Als Beispiel für solch ein Hapten kann Digoxigenin angeführt werden. Dem Fachmann sind über die angegebenen Beispiele auch noch weitere wohlbekannt.

Die abschließende Detektion der auf vorstehende beschriebene Weise markierten Zellen erfolgt im Durchflusszytometer. Die bei dieser Messung erhaltenen Messwerte werden in Form von Histogrammen oder Dotplots auf dem Computer visualisiert und erlauben die zuverlässige Aussage über Art und Menge der enthaltenen Bakterien.

Erfindungsgemäß wird ferner ein Kit zur Durchführung des erfindungsgemäßen Verfahrens bereitgestellt, der mindestens ein Nukleinsäuresondenmolekül zum spezifischen Nachweis eines Mikroorganismus, vorzugsweise bereits in der geeigneten Hybridisierungslösung enthält. Vorzugsweise sind weiterhin die geeignete Waschlösung, die Fixierungslösung sowie die Lösung zum Zellaufschluss und gegebenenfalls Reaktionsgefäße enthalten.

Wesentliche Vorteile des erfindungsgemäßen Verfahrens sind somit die sehr leichte Handhabung sowie weiterhin Schnelligkeit, Reproduzierbarkeit, Zuverlässigkeit und Objektivität, mit der der spezifische Nachweis von Mikroorganismen in einer Probe möglich ist.

Ein weiterer Vorteil besteht darin, dass nun erstmals auch für gram-positive Organismen das vorteilhafte Verfahren der in situ-Hybridisierung in Lösung durchgeführt werden kann. So können erstmals die miteinander kombinierten Vorteile der FISH und der Durchflusszytometrie für die Analyse gram-positiver Organismen genutzt werden.

Ein weiterer Vorteil ist die gegenüber dem Stand der Technik von 3 Stunden auf bevorzugt 1,5 Stunden verkürzte Hybridisierungsdauer.

Ein weiterer Vorteil ist die Spezifität des Verfahrens. Entscheidend ist hierbei, dass die Konzentration und das Volumen der verwendeten Waschlösung auf das Volumen der im vorausgehenden Schritt verwendeten Hybridisierungslösung abgestimmt werden. Direkt nach dem Vermischen der Hybridisierungslösung und der Waschlösung liegen die in der Waschlösung enthaltenen Chemikalien in der für die Spezifität der Nachweisreaktion erforderlichen Konzentration vor. Gemäß den Techniken des Standes der Technik muss zunächst die Hybridisierungslösung (z.B. durch einen Zentrifugationsschritt) entfernt werden, bevor die Waschlösung zugegeben werden kann. Hierbei sinkt die Temperatur des Reaktionsgemisches auf Raumtemperatur ab. Bei dieser niedrigen Temperatur binden die in der Hybridisierungsreaktion verwendeten Nukleinsäuresondenmoleküle dann unspezifisch auch in solchen Zellen, die nicht die exakten Zielstellen für die Nukleinsäuresondenmoleküle enthalten, sondern lediglich ähnliche Sequenzen. Beim abschließenden Detektionsschritt werden dann auch diese infolge der unspezifischen Bindung der Nukleinsäuresondenmoleküle markierten Nichtzielzellen detektiert. Ein falsch positives Ergebnis ist die Folge. Dagegen ist bei Anwendung des erfindungsgemäßen Verfahrens gewährleistet, dass die Temperatur während des gesamten Vorganges von Hybridisierung und Waschen konstant bleibt, wodurch erstmals sicher die Spezifität des Nachweisverfahrens gewährleistet ist.

Ein weiterer Vorteil ist die gegenüber dem Stand der Technik von 30 Minuten auf bevorzugt 15 Minuten verkürzte Dauer des Waschschritts.

Der mittels des erfindungsgemäßen Verfahrens nachzuweisende Mikroorganismus kann ein prokaryontischer oder eukaryontischer Mikroorganismus sein. In den meisten Fällen wird es erwünscht sein, einzellige Mikroorganismen nachzuweisen. Diese einzellige Mikroorganismen können auch in größeren Aggregaten, sogenannten Filamenten, vorliegen. Relevante Mikroorganismen sind hierbei vor allem Hefen, Algen, Bakterien oder Pilze.

Das erfindungsgemäße Verfahren kann vielfältig angewendet werden.

So können beispielsweise Umweltproben auf das Vorhandensein von Mikroorganismen untersucht werden. Diese Proben können hierzu aus Luft, Wasser oder aus dem Boden entnommen sein.

Ein weiteres Anwendungsgebiet für das erfindungsgemäße Verfahren ist die Kontrolle von Lebensmitteln. In bevorzugten Ausführungsformen werden die Lebensmittelproben aus Milch oder Milchprodukten (Joghurt, Käse, Quark, Butter, Buttermilch), Trinkwasser, Getränken (Limonaden, Bier, Säfte), Backwaren oder Fleischwaren entnommen.

Das erfindungsgemäße Verfahren kann weiter zur Untersuchung medizinischer Proben eingesetzt werden. Es ist für die Untersuchung von Gewebeproben, z.B. Biopsiematerial aus der Lunge, Tumor- oder entzündliches Gewebe, aus Sekreten wie Schweiß, Speichel, Sperma und Ausfluss aus der Nase, Harnröhre oder Vagina sowie für Urin- oder Stuhlproben geeignet.

Ein weiteres Anwendungsgebiet für das vorliegende Verfahren ist die Untersuchung von Abwässern, z.B. Belebtschlamm, Faulschlamm oder anaeroben Schlamm. Darüber hinaus ist es geeignet, Biofilme in industriellen Anlagen zu analysieren, sowie auch sich natürlicherweise bildende Biofilme oder bei der Abwasserreinigung bildende Biofilme zu untersuchen. Auch die Untersuchung pharmazeutischer und kosmetischer Produkte, z.B. Salben, Cremes, Tinkturen, Säfte etc. ist mit dem erfindungsgemäßen Verfahren möglich.

Das folgende Beispiel soll die Erfindung beschreiben, ohne sie einzuschränken.

### Beispiel:

Kombiniertes Verfahren zum spezifischen Nachweis von Mikroorganismen am Beispiel des Nachweises bierschädlicher Laktobazillen.

Die zu untersuchende Probe wird in geeigneter Weise 24 - 48 h kultiviert. Verschiedene hierzu geeignete Verfahren und Kultivierungsmedien sind dem Fachmann wohlbekannt. Ein Aliquot der Kultur (z.B. 2 ml) wird in ein geeignetes Reaktionsgefäß überführt und die enthaltenen Zellen durch Zentrifugation sedimentiert (4000 x g, 5 min, Raumtemperatur).

Anschließend wird ein geeignetes Volumen (bevorzugt 20 µl) der Fixierungslösung zugegeben und das offenen Reaktionsgefäß bei ≥ 37 °C inkubiert bis die Fixierungslösung vollständig verdunstet ist.

Anschließend wird ein geeignetes Volumen der Enzymlösung (bevorzugt 30 - 40 µl Lysozym [1 mg/ml H₂O]) zugegeben und die Probe 7 Minuten bei Raumtemperatur inkubiert.

Anschließend wird ein geeignetes Volumen (bevorzugt 90 -120 µl) 1,33-fach konzentrierter Hybridisierungslösung, enthaltend die markierten Nukleinsäuresondenmoleküle für den spezifischen Nachweis der bierschädlichen Laktobazillen, zugegeben und die Probe inkubiert (46 °C, 1,5 h).

Anschließend wird ein geeignetes Volumen 5-fach konzentrierter Waschlösung (bevorzugt 30 - 40 µl) zugefügt und die Probe für weitere 15 Minuten bei 46 °C inkubiert.

Anschließend wird die Probe zentrifugiert (4000 x g, 5 min, Raumtemperatur). Der Überstand wird verworfen und das Sediment in einem geeigneten Volumen gepufferter Phosphatlösung (bevorzugt 100 - 200 µl) aufgenommen.

Die so vorbereitet Probe wird jetzt am Durchflusszytometer (z. B. Microcyte, Optoflow, Norwegen) analysiert.

### Beispiel:

Kombiniertes Verfahren zum spezifischen Nachweis von Mikroorganismen am Beispiel des Nachweises von Laktobazillen.

### 1. Material

### 1.1 Verwendete Mikroorganismen

| Organismus | Stammbezeichnung | | Anzuchtbedingungen |
|---|---|---|---|
| *Lactobacillus brevis* | WSB | L32 | M11/ 30°C/ stehend - mikroaerophil |
| *Escherichia coli* | DSM | 30083 | M1/ /37°C/ geschüttelt 100 Upm - aerob |
| *Pediococcus damnosus* | TUM | 618 | M231/ /30°C/ stehend - mikroaerophil |
| *Salmonella cholerasuis* ssp. *cholerasuis* | DSM | 554 | M1/ 37°C/ geschüttelt 100 Upm - aerob |
| *Staphylococcus aureus* ssp. *aureus* | DSM | 1104 | M1/ 37°C/ geschüttelt 100 Upm - aerob |

Die Bakterienstämme mit der Bezeichnung DSM sind von der DSMZ (Deutsche Sammlung von Mikroorganismen und Zellkulturen GmbH, Braunschweig, Deutschland) erhältlich. Die Stämme WSB L32 und TUM 618 sind Stämme aus der Laborsammlung der WSB (Faculty of Technology of Brewery I, Freising-Weihenstephan, Deutschland) und der Technischen Universität München TUM (Faculty of Microbiology, Freising-Weihenstephan, Deutschland).

### 1.2 Verwendete Medien

### Medium 11: MRS MEDIUM

| | |
|---|---|
| Casein-Pepton, tryptisch verdaut | 10,00 g |
| Meat-Extract | 10,00 g |
| Yeast-Extract | 5,00 g |
| Glucose | 20,00 g |
| Tween 80 | 1,00 g |
| K₂HPO₄ | 2,00 g |
| Na-Acetat | 5,00 g |
| (NH₄)₂ Citrat | 2,00 g |
| MgSO₄ x 7H₂O | 0,20 g |
| MnSO₄ x H₂O | 0,05 g |
| destilliertes Wasser ad | 1000,00 ml |

Einstellen des pH-Wertes auf 6,2 - 6,5.

### Medium 231: PEDIOCOCCUS DAMNOSUS MEDIUM

Einstellen des pH-Wertes von Medium 11 (MRS-Medium) auf pH 5,2.

### Medium 1: NUTRIENT Medium

| | |
|---|---|
| Pepton | 5,0 g |
| Meat-Extract | 3,0 g |
| Destilliertes Wasser | ad 1000,0 ml |

Einstellen des pH-Werts auf 7,0.

Alle vorstehend genannten Medien, die für die Anzucht von Bakterien verwendet wurden, sind von der DSMZ (Deutsche Sammlung von Mikroorganismen und Zellkulturen GmbH, Braunschweig, Deutschland) kommerziell erhältlich.

### 1.3 Verwendete Lösungen

### Hybridisierungslösung (1,5-fach konzentriert)

| **BESTANDTEIL** | **MENGE** | **ENDKONZENTRATION 1,5-fach** | **ENDKONZENTRATION 1-fach** |
|---|---|---|---|
| NaCl-Lösung (5 Mol/l) | 2,7 ml | 1350 mMol/l | 900 mMol/l |
| Tris-HCl-Puffer (1 Mol/l) | 300 µl | 30 mMol/l | 20 mMol/l |
| Aqua | 1,7 ml | --- | --- |
| SDS-Lösung (10%) | 7,5 µl | 0,015 % | 0,01% |
| Formamid | 5,3 ml | 52,5 % | 35% |
| **Endvolumen:** | **10 ml** | **---** | **---** |

### Waschlösung (4-fach konzentriert)

| **BESTANDTEIL** | **MENGE** | **ENDKONZENTRATION 4-fach** | **ENDKONZENTRATION 1-fach** |
|---|---|---|---|
| Tris-Puffer (1 Mol/l) | 4 ml | 80 mMol/l | 20 mMol/l |
| NaCl-Lösung (5 Mol/l) | 2,8 ml | 280 mMol/l | 70 mMol/l |
| EDTA-Lösung (0,5 Mol/l) | 2 ml | 20 mMol/l | 5 mMol/l |
| Aqua | 41,0 ml | --- | --- |
| **Endvolumen:** | **50,0 ml** | **---** | **---** |

### 2. Durchführung:

Die Anreicherung der zu untersuchenden Bakterienkulturen wurde wie unter dem Punkt "1.1 Verwendete Mikroorganismen" angegeben, durchgeführt. Anschließend wurde ein Aliquot der Kultur (1 - 2 ml) in ein Reaktionsgefäß überführt und die enthaltenen Zellen wurden durch Zentrifugation sedimentiert (4000 x g, 5 min, Raumtemperatur).

Der Überstand wurde verworfen und zum Zellpellet wurden 15 µl der Fixierungslösung (99,8 % EtOH) zugegeben und das offene Reaktionsgefäß bei 46 °C inkubiert, bis die Fixierungslösung vollständig verdunstet war.

Anschließend wurden 40 µl der Enzymlösung (Lysozym [1 mg/ml H₂O]) zugegeben und die Probe 7 Minuten bei Raumtemperatur inkubiert.

Anschließend wurden 80 µl 1,5-fach konzentrierte Hybridisierungslösung, enthaltend ein Cy5-markiertes Nukleinsäuresondenmolekül (Lgc-354a 5' - TGGAAGATTCCCTACTGC - 3') zugegeben und die Probe inkubiert (46 °C, 1,5 h).

Anschließend wurden 40 µl 4-fach konzentrierte Waschlösung zugefügt und die Probe für weitere 15 Minuten bei 46 °C inkubiert.

Abschließend wurde die Probe zentrifugiert (4000 x g, 5 min, Raumtemperatur). Der Überstand wurde verworfen und das Sediment in einem geeigneten Volumen gepufferter Phosphatlösung (bevorzugt 100 - 200 µl) aufgenommen. Dieser letzte Zentrifugationsschritt ist optional; alternativ kann die Probe auch gänzlich ohne Zentrifugationsschritt direkt im Anschluss an den Waschschritt vermessen werden.

Die so vorbereitete Probe wurde am Durchflusszytometer (Microcyte, Optoflow, Norwegen) unter Verwendung der MC2200 Software (Fa. Optoflow, Norwegen) analysiert. Desweiteren wurde die Software WinMDI 2.8 **(Win**dows **M**ultiple **D**ocument **I**nterface for Flow Cytometry), ein unter http://facs.scripps.edu/software.html frei erhältliches Programm, zur graphischen Nachbearbeitung der Messdaten verwendet.

### Alternative:

Alternativ wurde nach dem Zentrifugieren des Probenaliquots der Überstand verworfen und zum Zellpellet wurden 5 µl der Enzymlösung (Lysozym [1 mg/ml H₂O]) zugegeben und die Probe 7 Minuten bei Raumtemperatur inkubiert.

Anschließend wurden 10 µl der Fixierungslösung (99,8% EtOH) zugegeben und das offene Reaktionsgefäß bei 46 °C inkubiert bis die Fixierungslösung vollständig verdunstet war.

In diesem Fall erfolgte der nachfolgend durchgeführte Hybridisierungsschritt unter Zugabe von 120 µl 1-fach konzentrierter Hybridisierungslösung (anstelle von 80 µl 1,5-fach konzentrierter Lösung). Alle weiteren Schritte blieben unverändert.

### 3. Ergebnisse

Im Gegensatz zur visuellen Überprüfung am Mikroskop besteht am Durchflusszytometer nur sehr eingeschränkt die Möglichkeit, unspezifische Bindungen oder Artefakte von einem spezifischen Signal zu unterscheiden, sofern diese Ereignisse in einem ähnlichen Größenverhältnis auftreten.

Deshalb ist es unumgänglich einen Schwellenwert oder ein Detektionslimit festzusetzen. Messwerte unterhalb dieses Limits werden als Hintergrundrauschen interpretiert; Messwerte oberhalb dieses Wertes werden als positiver Befund bewertet.

Dieses Detektionslimit wurde bestimmt mittels Vermessung von Reinstwasser, 1 x PBS, Zellen, die ohne Sonde der Hybridisierungsprozedur unterzogen wurden und Zellen, die mit einer nichtbindenden Oligonukleotidsonde hybridisiert wurden, und lag bei 9 x 10³ counts/ml.

### 3.1 Negative Befunde

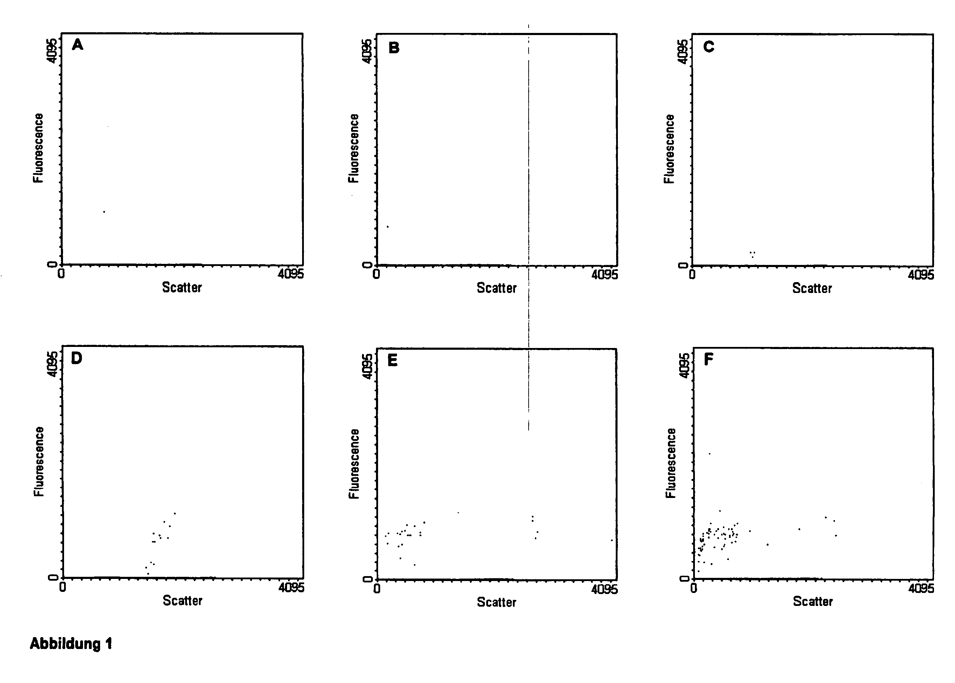

Abbildung 1 zeigt die mit Nicht-Zielorganismen der verwendeten Sonde erhaltenen Ergebnisse. Die erhaltenen Werte lagen zwischen 1,0 x 10³ und 3,1 x 10³ counts/ml (mit Zentrifugationsschritt nach dem Waschen, Abb. 1A bis C) bzw. zwischen 4,5 x 10³ und 6,7 x 10³ counts/ml (ohne Zentrifugationsschrit nach dem Waschen, Abb. 1D bis F) und lagen damit deutlich unterhalb des Detektionslimits. Mit finalem Zentrifugationsschritt lagen die Werte niedriger als ohne diesen Schritt, aber auch ohne finalen Zentrifrugationsschritt die Analyse erfolgreich durchgeführt werden.

### 3.2 Positive Befunde

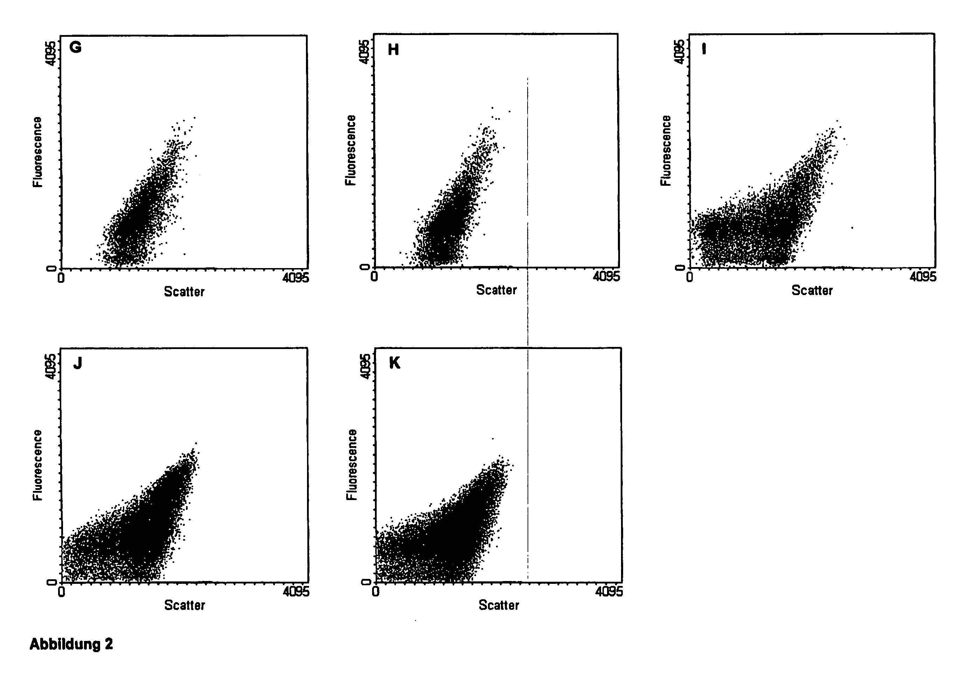

Abbildung 2 zeigt die mit Zielorganismen der verwendeten Sonde erhaltenen Ergebnisse. Die erhaltenen Werte lagen sämtlich deutlich oberhalb des Detektionslimits. Die bei der Analyse von Rein- und Mischkulturen (Abbildungen 2G - L) erhaltenen Messwerte waren stabil und miteinander vergleichbar. Auch die Messwerte für unterschiedliche Zellmengen (Verarbeitung von 1 ml bzw. 2 ml einer Kultur) zeigten sowohl für *Lactobacillus brevis* als auch für *Pediococcus damnosus* eine gute Korrelation.

Die Vermessung von *Lactobacillus brevis-* (s. Abb. 2I, J und L) und *Pediococcus damnosus-* (s. Abb. 2G u. H) Zellen erbrachte nicht nur reproduzierbare Messwerte, sondern auch unterschiedliche, von der Morphologie der Zellen abhängige Verteilungen der Einzelereignisse.

Die unterschiedliche Form der erhaltenen Plots kann primär mit der unterschiedlichen Morphologie (*P. damnosus* = Kokken und *L. brevis* = Stäbchen) erklärt werden. Zusätzlich wird auch die Homo- bzw. Heterogenität einer Kultur in der unterschiedlichen Darstellungsart verdeutlicht. So wird die aus etwa gleich großen und gleichförmigen Zellen bestehende Kultur von *P. damnosus* kegelförmig dargestellt (s. Abb. 2G u. H). Die Verteilung der Einzelmessergebnisse der sehr heterogenen Kultur von *L. brevis,* die aus Zellen von sehr unterschiedlicher Morphologie und Größe (kurze, lange, z.T. filamentartige Strukturen annehmende Stäbchen) wird in der Form, ähnlich der eines Dreiecks, dargestellt (s. Abb. 2I, J und L). Die in Abbildung K dargestellte Verteilung der Einzelmessereignisse einer Mischung aus *L. brevis* und *P. damnosus* Kultur, birgt beide unterschiedlichen Verteilungsformen in einer Messung.

### Erläuterung der Abbildungen:

### Abbildung 1: Ergebnisse einiger Versuche mit negativem Befund

**A**: Density plot von *Staphylococcus aureus* Zellen gefärbt mit der Sonde Lgc-354a; Messwert: 1,3 x 10² counts/ml **B**: Density plot von *Escherichia coli*-Zellen gefärbt mit der Sonde Lgc-354a; Messwert: 1,0 x 10³ counts/ml **C**: Density plot von *Salmonella cholerasuis-*Zellen gefärbt mit der Sonde Lgc-354a; Messwert: 3,1 x 10³ counts/ml **D**: Density plot von *Staphylococcus aureus* Zellen gefärbt mit der Sonde Lgc-354a; Messwert: 4,5 x 10³ counts/ml **E**: Density plot von *Escherichia coli-*Zellen gefärbt mit der Sonde Lgc-354a; Messwert: 5,2 x 10³ counts/ml **F**: Density plot von *Salmonella cholerasuis-*Zellen gefärbt mit der Sonde Lgc-354a; Messwert: 6,7 x 10³ counts/ml

### Abbildung 2: Ergebnisse einiger Versuche mit positivem Befund

**G**: Density plot von 1 ml *Pediococcus damnosus-*Zellen hybridisiert mit der Sonde Lgc-354a; Messwert 5,2 x 10⁵ counts/ml **H**: Density plot von 2 ml *P. damnosus-*Zellen hybridisiert mit der Sonde Lgc-354a; Messwert 9,8 x 10⁵ counts/ml **I**: Density plot von 1 ml *Lactobacillus brevis* Zellen detektiert mit der Sonde Lgc-354a; Messwert 6,16 x 10⁵ counts/ml **J:** Density plot von 2 ml *L. brevis* Zellen detektiert mit der Sonde Lgc-354a; Messwert: 1,27 x 10⁶ counts/ml **K**: Density plot einer Mischung von 1 ml *P. damnosus-*Zellen und 1 ml *L. brevis* Zellen gefärbt mit der Sonde Lgc-354a; Messwert 1,6 x 10⁶ **L**: Density plot von 1 ml *L. brevis* Zellen detektiert mit der Sonde Lgc-354a; Messwert 6,34 x 10⁵ counts/ml. **G** bis **K**: mit Zentrifugation nach dem Waschschritt; **L**: ohne Zentrifugation nach dem Waschschritt.

## Patentansprüche

1. Verfahren zum Nachweis von Mikroorganismen in einer Probe durch *in situ-*Hybridisierung und Durchflusszytometrie, umfassend die Schritte:
a) Fixieren der in der Probe enthaltenen Mikroorganismen unter Verwendung eines Fixierungsmittels,
i) Trocknen der Probe und **dadurch** Entfernen des Fixierungsmittels,
b) Inkubieren der fixierten Mikroorganismen mit in einer Hybridisierungslösung enthaltenen Nukleinsäuresondenmolekülen, um eine Hybridisierung herbeizuführen (= Hybridisierungsschritt),
c) Zugeben einer Waschlösung zu den fixierten und mit den Nukleinsäuresondenmolekülen inkubierten Mikroorganismen (= Waschschritt),
d) Detektieren der Mikroorganismen mit hybridisierten Nukleinsäuresondenmolekülen mittels Durchflusszytometrie,
wobei zwischen dem Hybridisierungsschritt b) und dem Waschschritt c) kein Entfernen der Hybridisierungslösung stattfindet.

2. Verfahren nach Anspruch 1, weiter umfassend zwischen dem Trocknungsschritt i) und dem Hybridisierungsschritt b) den Schritt:
ii) Aufschließen der fixierten Mikroorganismen.

3. Verfahren nach Anspruch 1 oder 2, wobei der Mikroorganismus eine Hefe, ein Bakterium, eine Alge oder ein Pilz ist.

4. Verfahren nach Anspruch 2, wobei es sich bei den Mikroorganismen um gram-positive Bakterien handelt.

5. Verfahren nach einem der vorangehenden Ansprüche, wobei die Nukleinsäuresondenmoteküle kovalent mit einem detektierbaren Marker verbunden sind und der detektierbare Marker ausgewählt ist aus der Gruppe, bestehend aus Fluoreszenzmarkern, Chemolumineszenzmarkern, radioaktiven Markern, enzymatisch aktiven Gruppen, Haptenen, durch Hybridisierung nachweisbaren Nukleinsäuren.

6. Verfahren nach einem der vorangehenden Ansprüche, wobei die Dauer des Waschschritts weniger als 30 Minuten beträgt.

7. Verfahren nach Anspruch 6, wobei die Dauer des Waschschritts nicht mehr als 20 Minuten, bevorzugt nicht mehr als 15 Minuten beträgt.

## Claims

1. Method for identifying microorganisms in a sample by *in situ* hybridisation and flow cytometry, comprising the steps of:
a) fixing the microorganisms contained in the sample using a fixing agent,
i) drying the sample and thereby removing the fixing agent,
b) incubating the fixed microorganisms with nucleic acid probe molecules contained in a hybridisation solution, in order to bring about hybridisation (= hybridisation step),
c) adding a wash solution to the microorganisms that have been fixed and have been incubated with the nucleic acid probe molecules (= washing step),
d) detecting the microorganisms with hybridised nucleic acid probe molecules by means of flow cytometry, whereby no removal of the hybridisation solution takes place between the hybridisation step b) and the washing step c).

2. Method according to Claim 1, further comprising, between the drying step i) and the hybridisation step b), the step of:
ii) breaking down the fixed microorganisms.

3. Method according to Claim 1 or 2, whereby the microorganism is a yeast, a bacterium, an alga or a fungus.

4. Method according to Claim 2, whereby the microorganisms are gram-positive bacteria.

5. Method according to one of the preceding claims, whereby the nucleic acid probe molecules are connected covalently to a detectable marker and the detectable marker is selected from the group consisting of fluorescence markers, chemoluminescence markers, radioactive markers, enzymatically active groups, haptens, nucleic acids identifiable by hybridisation.

6. Method according to one of the preceding claims, whereby the washing step takes less than 30 minutes.

7. Method according to Claim 6, whereby the washing step takes no more than 20 minutes, preferably no more than 15 minutes.

## Revendications

1. Procédé de détection de micro-organismes dans un échantillon par hybridation *in situ* et cytométrie de flux, comprenant les étapes consistant à:
a) fixer les micro-organismes contenus dans l'échantillon en utilisant un agent de fixation,
i) sécher l'échantillon et éliminer ainsi l'agent de fixation,
b) incuber les micro-organismes fixés avec des molécules de sondes d'acides nucléiques contenues dans une solution d'hybridation, pour effectuer une hybridation (= étape d'hybridation),
c) ajouter une solution de lavage aux micro-organismes fixés et incubés avec les molécules de sondes d'acides nucléiques (= étape de lavage),
d) détecter par cytométrie de flux les micro-organismes avec les molécules de sondes d'acides nucléiques hybridées,
dans lequel aucun enlèvement de la solution d'hybridation n'a lieu entre l'étape d'hybridation b) et l'étape de lavage c).

2. Procédé selon la revendication 1, comprenant en plus, entre l'étape de séchage i) et l'étape d'hybridation b), l'étape consistant à :
ii) lyser les micro-organismes fixés.

3. Procédé selon la revendication 1 ou la revendication 2, dans lequel le micro-organisme est une levure, une bactérie, une algue ou un champignon.

4. Procédé selon la revendication 2, dans lequel le micro-organisme est une bactérie à Gram positif.

5. Procédé selon l'une des revendications précédentes, dans lequel les molécules de sondes d'acides nucléiques sont liées de manière covalente à un marqueur détectable, et le marqueur détectable est choisi dans le groupe constitué par des marqueurs fluorescents, des marqueurs chimioluminescents, des marqueurs radioactifs, des groupes enzymatiquement actifs, des haptènes, des acides nucléiques détectables par hybridation.

6. Procédé selon l'une des revendications précédentes, dans lequel la durée de l'étape de lavage est inférieure à 30 minutes.

7. Procédé selon la revendication 6, dans lequel la durée de l'étape de lavage n'est pas supérieure à 20 minutes, de préférence non supérieure à 15 minutes.
